# EUROPEAN PATENT APPLICATION

(11) **EP 4 804 195 A1**
(43) Date of publication of application: **09.09.2026**
(21) Application number: 25170055.5
(22) Date of filing: 11.04.2025
(51) Int. Cl.: G16B 20/00, G16B 20/20, G16B 40/20, G16B 50/10

(54) **METHOD FOR GENETIC VARIANT ANALYSIS BASED ON RNA SAMPLES**

(30) Priority: 03.03.2025 US 202563766175 P
(71) Applicant: Genialis, Inc., Boston, 02115-3153 (US)
(72) Inventor: Kokosar, Janez, Boston, 02111 (US); Otonicar, Jan, Boston, 02111 (US); Levstek, Marcel, Boston, 02111 (US); Lovse, Anze, Boston, 02111 (US); Lustrik, Roman, Boston, 02111 (US); Zganec, Matjaz, Boston, 02111 (US); Stajdohar, Miha, Boston, 02111 (US); Urh, Kristian, Boston, 02111 (US); Ausec, Luka, Boston, 02111 (US)
(74) Representative: Maiwald GmbH

(57) **Abstract**

Disclosed is a method for genetic variant analysis based on RNA samples, the method comprising reading variant calling information of an RNA sample for a plurality of variant sites, selecting a subset of the plurality of variant sites based on a predetermined gene set and independently of whether the variant calling information comprises, for the respective variant site identified by the predetermined gene set, a predicted alteration event for the variant site in the RNA sample; for the subset of the plurality of variant sites: retrieving annotation data by matching at least some of the variant sites of the subset of variant sites with predetermined variant sites from a predetermined dataset, the predetermined dataset comprising annotation data associated with the predetermined variant sites, providing, for each matched variant site of the subset of variant sites, an annotation comprising an identification of the variant site and corresponding annotation data, and predicting, for one or more variant sites of the subset of variant sites, a presence and/or an absence and/or an undetermined presence/absence of an alteration event at the respective variant site in the genome associated with the RNA sample based on the variant calling information; and providing analysis data comprising the annotations of the variant sites of the subset of variant sites and/or data derived therefrom and the predicted presence and/or absence and/or undetermined presence/absence of an alteration event at the respective variant site in the genome associated with the RNA sample.

## Description

### BACKGROUND OF THE INVENTION

The present disclosure relates to a computer-implemented method for genetic variant analysis based on RNA samples, corresponding computer program product, computer-readable medium, and system, as well as the use of an annotation for analyzing an RNA sample.

Present methods for genetic variant analysis based on RNA samples face different challenges, including inaccuracies in the prediction and/or analysis of the results provided by standard variant callers.

The present disclosure aims to address these challenges and to provide an improved method for genetic variant analysis based on RNA samples, particularly allowing for improved prediction and/or analyzing accuracy.

### BRIEF SUMMARY OF THE INVENTION

The present disclosure provides a method, use, system, computer program product, and computer-readable medium according to the independent claims. Various additional embodiments are provided by the dependent claims.

The present disclosure provides a computer-implemented method for genetic variant analysis based on RNA samples, the method comprising reading variant calling information of an RNA sample for a plurality of variant sites. The method comprises selecting a subset of the plurality of variant sites based on a predetermined gene set and independently of whether the variant calling information comprises, for the respective variant site identified by the predetermined gene set, a predicted alteration event for the variant site in the RNA sample. The method comprises, for the subset of the plurality of variant sites, retrieving annotation data by matching at least some of the variant sites of the subset of variant sites with predetermined variant sites from a predetermined dataset, the predetermined dataset comprising annotation data associated with the predetermined variant sites, and providing, for each matched variant site of the subset of variant sites, an annotation comprising an identification of the variant site and corresponding annotation data. The method also comprises predicting, for one or more variant sites of the subset of variant sites, a presence and/or an absence and/or an undetermined presence/absence of an alteration event at the respective variant site in the genome associated with the RNA sample based on the variant calling information. The method further comprises providing analysis data comprising the annotations of the variant sites of the subset of variant sites and/or data derived therefrom and the predicted presence and/or absence and/or undetermined presence/absence of an alteration event at the respective variant site in the genome associated with the RNA sample.

According to another aspect, the present disclosure provides a computer-implemented method for genetic variant analysis based on RNA samples, the method comprising reading variant calling information of an RNA sample for a plurality of variant sites. The method comprises selecting a subset of the plurality of variant sites based on a predetermined gene set and independently of whether the variant calling information comprises, for the respective variant site identified by the predetermined gene set, a predicted alteration event for the variant site in the RNA sample. The method comprises, for the subset of the plurality of variant sites, retrieving annotation data by matching at least some of the variant sites of the subset of variant sites with predetermined variant sites from a predetermined dataset, the predetermined dataset comprising annotation data associated with the predetermined variant sites, and providing, for each matched variant site of the subset of variant sites, an annotation comprising an identification of the variant site and corresponding annotation data. The method further comprises providing analysis data comprising the annotations of the variant sites of the subset of variant sites and/or data derived therefrom.

According to another aspect, the present disclosure provides a computer-implemented method for genetic variant analysis based on RNA samples, the method comprising reading variant calling information of an RNA sample for a plurality of variant sites. The method comprises selecting a subset of the plurality of variant sites based on a predetermined gene set and independently of whether the variant calling information comprises, for the respective variant site identified by the predetermined gene set, a predicted alteration event for the variant site in the RNA sample. The method comprises, predicting, for one or more variant sites of the subset of variant sites, a presence and/or an absence and/or an undetermined presence/absence of an alteration event at the respective variant site in the genome associated with the RNA sample based on the variant calling information. The method further comprises providing analysis data comprising the predicted presence and/or absence and/or undetermined presence/absence of an alteration event at the respective variant site in the genome associated with the RNA sample.

As explained above, the present disclosure provides a method for genetic variant analysis based on RNA samples. The method comprising reading variant calling information of an RNA sample for a plurality of variant sites.

The term variant may refer to changes in the nucleotide sequence, different from the original (also called wild type, *wf*). Some of these differences may be inherited (i.e. germline variants), and some may be acquired (i.e. somatic variants).

Variants can be of different types, e.g. single nucleotide variants, structural variants, or copy number variants.

Accordingly, the term variant includes single nucleotide variants and short insertions/deletions (short INDELS). The term "single nucleotide variants" used herein refers to a variation in a single nucleotide in the genome (deletion, replacement or addition of a single nucleotide at a specific site).

Short insertions/deletions (short INDELS) refer to alterations where single nucleotides or a short sequence of nucleotides is inserted or deleted from the original sequence.

In an example, the term variant may refer to single nucleotide variants (SNV), such as single nucleotide polymorphism (SNP), and/or short INDELS.

A variant site may be a site that may contain an alteration event or not; It is a site of interest that contains an alteration event or there is a likelihood that an alteration event could occur. Variant site may refer to a specific base position in a specific chromosome. The term variant may include somatic and germline variants.

RNA samples may be samples comprising at least RNA. However, this does not preclude RNA samples also including other materials. The term RNA sample may refer to a biological sample, such as tissue and/or liquid sample. In particular, it may refer to a mammalian sample, such as a sample derived from the human body (e.g. blood, salvia, tissue).

The skilled person understands that if the term "RNA sample" is used herein, the RNA sequencing data derived from sequencing the RNA sample is entailed.

In other words, sequencing an RNA sample yields corresponding RNA sequencing data of the RNA sample.

The skilled person also understands that RNA sequencing data of an RNA sample is obtained by analyzing the RNA sample.

Accordingly, variant calling information of an RNA sample refers to information extracted from RNA sequencing data of an RNA sample obtained by analyzing the RNA sample.

The variant calling information, which is read according to the method of the present disclosure, may comprise and/or be derived from the RNA sequencing data of the RNA sample.

The inventive method allows to retrieve based on the analysis of the variant calling information of the RNA sample to retrieve information on the genome (DNA) associated with the RNA sample, i.e. whether there are alteration events / variants and/or interpreting the presence of said alteration events / variants, for example in terms of its effects or impact, such as pathogenicity.

In the present disclosure, variant calling may refer to the determination/prediction for the presence or absence of an alteration event at a variant site.

Variant calling information of an RNA sample may comprise or be derived from information obtained by known variant calling methods. For example, variant calling information may be comprised in or derived from a VCF file provided by a known variant caller.

Variant calling information may comprise one or more predicted alteration events and/or non-alteration events for a plurality of variant sites in the RNA sample.

In particular, variant calling information may comprise information identifying a variant site with a predicted alteration event. In addition, variant calling information may comprise information indicating a confidence that there is an alteration event at the identified variant site or information indicating a confidence that no alteration event is present in spite of an alteration event being predicted.

Reading variant calling information may comprise receiving and/or retrieving variant calling information. Such information may have been obtained by variant calling. A variant caller may provide the variant calling information directly and/or store the variant calling information in a memory device, in which case reading variant calling information may comprise retrieving the stored variant calling information from the memory device.

As mentioned above, a subset of the plurality of variant sites is selected. That is, there is a plurality of variant sites that could, in theory, be analyzed for an alteration event. This can be considered a set of variant sites. The subset may have a lower number of variant sites than the set of variant sites.

The subset is selected based on a predetermined gene set. Thus, for example, only variant sites located on genes of the predetermined gene set are selected to become part of the subset of variant sites. The predetermined gene set may also be referred to as a panel of genes. As described below, the predetermined gene set may be application specific, e.g. dependent on a tissue or pathology the analysis aims at. The method may comprise selecting the gene set among a plurality of candidate gene sets and/or creating the gene set.

The subset is also selected independently of whether the variant calling information comprises, for the respective variant site identified by the predetermined gene set, a predicted alteration event for the variant site in the RNA sample. The subset may comprise variant sites for which the variant calling information predicted an alteration event and may also comprise variant sites for which the variant calling information does not predict an alteration event. The selection of the subset and, accordingly, the analysis data, thus, is not limited to variant sites for which the variant calling information predicted an alteration event. This allows for higher accuracy of the outcome of the analysis, as (possibly false) negatives in the variant calling information will not be disregarded by default. Instead, the method of the present disclosure allows for a more nuanced and accurate analysis of all variant sites of interest as identified in the predetermined gene set. This is not the case in known methods of variant calling from RNA sequencing data.

As explained above, different steps may be carried out for the subset of the plurality of variant sites.

In one aspect, the method comprises retrieving annotation data by matching at least some of the variant sites of the subset of variant sites with predetermined variant sites from a predetermined dataset, the predetermined dataset comprising annotation data associated with the predetermined variant sites.

The predetermined dataset, particularly the annotation data, may, for example, comprise, for each respective predetermined variant site, a variant site identifier, a chromosome where the variant site is located, a variant position in the reference genome, e.g. on the chromosome, an impact level of an alteration at the variant site (e.g. based on the type of alteration), a variant effect of an alteration the variant site (e.g. pathogenicity), a gene affected by an alteration at the variant site, a gene feature affected by an alteration at the variant site, a protein-level change or amino acid change caused by an alteration at the variant site, presence and/or frequency of an alteration event at the variant site in population/demographic, and/or other clinical effect metadata.), a variant effect of an alteration the variant site (e.g. pathogenicity), a gene affected by an alteration at the variant site, a gene feature affected by an alteration at the variant site, a protein-level change or amino acid change caused by an alteration at the variant site, presence and/or frequency of an alteration event at the variant site in population/demographic, and/or other clinical effect metadata.

The predetermined dataset may, for example, be a database. The predetermined data set may be application-specific, for example specific for the tissue and/or pathology to be analyzed. The method may comprise selecting, among a plurality of datasets, the predetermined dataset.

The selection of the predetermined variant sites that are included in the predetermined dataset may be application-specific, for example specific for the tissue and/or pathology to be analyzed. For example, a variant site may be selected as predetermined variant site of the predetermined data set in case an alteration event at said variant site is potentially relevant for the application at hand, e.g. tissue and/or pathology.

The matching may comprise determining, for a respective variant site of the subset of variant sites, determining whether the predetermined variant sites comprise said respective variant site. For example, this may be accomplished using an identifier of the variant site, wherein an identifier may be any information that unambiguously identifies the variant site. In case the predetermined variant sites comprise the respective variant site, this may be considered a match. In other words, a matching predetermined variant site is present in the predetermined dataset. A variant site having a matching predetermined variant site may be referred to as matched variant site.

The method may further comprise providing, for each matched variant site of the subset of variant sites, an annotation comprising an identification of the variant site and corresponding annotation data.

The corresponding annotation data may comprise or be derived from annotation data associated with the matching predetermined variant site. The corresponding annotation data may be obtained by retrieving and optionally further processing the annotation data from the predetermined data set.

The annotation may comprise, e.g. in addition to annotation data from the predetermined data set or data derived therefrom, at least part of the variant calling data, for example a read depth and/or a confidence indicator associated with the variant calling, and/or data indicative of data processing steps, for example data indicative of whether one or more filter criteria are met, which may be referred to as a filter status.

An annotation may, for example, be provided as a data object, which may be referred to as ANN object.

The annotation may be used for different purposes, for example for applying filters and/or preparing data for processing by subsequent automated processing. Optionally, at least part of the annotation may be used in determining a presence, absence, and/or undetermined presence/absence of an alteration event at the variant site.

As will be explained in more detail below, the predetermined variant sites may not necessarily comprise a matching predetermined variant site for all of the variant sites of the subset of variant sites. A variant site that does not have a matching predetermined variant site may be annotated using any known annotation process to obtain an annotation, such as querying the variant site against a public database such as ClinVar. Optionally, the predetermined dataset may then be extended on the basis of this annotation process.

It will be understood that the method described above will allow for arriving at annotations for the sample in an efficient manner. Particularly, it is not necessary to annotate each variant site, but results of previous annotation processes and/or other data sources may be leveraged via the matching step.

Accordingly, annotations can be provided even for a larger set of variant sites efficiently, allowing for obtaining, at similar efficiency, a more complete picture of the sample.

According to another aspect, which may optionally be combined with the first aspect, the method of the present disclosure comprises predicting, for one or more variant sites of the subset of variant sites, a presence and/or an absence and/or an undetermined presence/absence of an alteration event at the respective variant site in the genome associated with the RNA sample based on the variant calling information.

The prediction is made based on the variant calling information. That is, at least part of the variant calling information may be used as an input for the prediction. The variant calling information may comprise, for one or more variant sites, a prediction of an alteration event at the respective variant site. The variant calling information may additionally comprise, for one or more variant sites, information about the sequencing coverage at the variant site. The prediction, optionally together with a confidence provided by or derived from the variant calling data may be used for predicting a presence and/or an absence and/or an undetermined presence/absence of an alteration event at the respective variant site.

The predicting may, in particular, comprise determining a confidence score. The confidence score may be, for each variant site for which a prediction is made, a level of confidence in alteration even at the respective variant site. The confidence score may be output together with the prediction of an event or non-event so as to indicate the presence and/or absence and/or undetermined presence/absence, e.g. as a pair of values. Alternatively, the presence and/or absence and/or undetermined presence/absence may be derived from the confidence score, for example based on a respective confidence interval and/or threshold associated with the presence, absence or undetermined presence/absence. For example, it may be determined whether the confidence score is in an interval associated with the presence, an interval associated with the absence, or an interval associated with the undetermined presence/absence of an alteration event. Yet alternatively, an event or non-event probability may be derived from the confidence score and the presence and/or absence and/or undetermined presence/absence may be derived from the probability, for example based on a respective probability interval and/or threshold associated with the presence, absence or undetermined presence/absence. For example, it may be determined whether the probability is in an interval associated with the presence, an interval associated with the absence, or an interval associated with the undetermined presence/absence of an alteration event. This will be described in more detail below.

The prediction of a presence and/or an absence and/or an undetermined presence/absence of an alteration event at the respective variant site in the genome associated with the RNA sample may be made independently of whether the variant calling information comprises, for the respective variant site identified by the predetermined gene set, a predicted alteration event for the variant site in the RNA sample. For example, a prediction may be made for a variant site also in case the variant calling information did not predict an alteration event at said variant site.

The prediction of a presence and/or an absence and/or an undetermined presence/absence of an alteration event at the respective variant site in the genome associated with the RNA sample may optionally take into account information comprised in or derived from the above-described annotation. For example, the annotation may comprise information indicating a prevalence of an alteration event for a population/demographic, e.g. a presence and/or frequency of an alteration event at the variant site in a population/demographic. A high prevalence may increase confidence in determining a presence of an alteration event.

It will be understood from the above that the method of the present disclosure allows for more accurate results of the analysis, as, in addition to a high-accuracy positive prediction for an alteration event predicted in the variant calling information, it also allows for high-accuracy predictions for variant sites where an alteration event is not predicted in the variant calling information.

The method of the present disclosure comprises providing analysis data. The analysis data comprising the annotations of the variant sites of the subset of variant sites and/or data derived therefrom and/or the predicted presence and/or absence and/or undetermined presence/absence of an alteration event at the respective variant site in the genome associated with the RNA sample.

Analysis data may be used, for example, for further processing, such as by machine learning models. Analysis data may also be output to a user.

The analysis data may be or comprise a final annotation. In an example, the final annotation may be a modified annotation obtained by modifying the (initial) annotation described above at least by adding the predicted presence and/or absence and/or undetermined presence/absence of an alteration event at the respective variant site in the genome associated with the RNA sample. Alternatively or in addition, the final annotation may be obtained by modifying the annotation described above by adding data from data sources after initially generating the annotation and/or by removing data after initially generating the annotation, e.g. based on filtering and or selection steps, such as based on pathogenicity and/or confidence score. Multiple modification steps are conceivable, each resulting in a respective modified annotation.

In case a confidence score is determined, the analysis data, particularly the final annotation, may comprise the confidence score.

According to the present disclosure, the method may comprise filtering of variant sites from the plurality of variant sites based on variant site quality scores and/or mean exon coverage, particularly prior to the annotating.

A filtering of variant sites may be carried out once or several times with different filter criteria. For example, one or more initial filtering steps may be carried out prior to the matching described above. Initial filtering steps may, for example, comprise filtering variant as part of a selection of the subset of variant sites. As another example, intermediate filtering steps may be carried out after providing the annotation and prior to providing the analysis data, particularly prior to creating a final annotation. As yet another example, downstream filtering steps may be carried out after providing the analysis data, for example after providing the final annotation.

Filtering may comprise filtering for quality criteria, in particular criteria associated with allele depth, coverage, confidence, fisher strand bias, or the like. This filtering may be carried out by determining whether respective quality criteria are met based on the variant calling data. In particular, this type of filtering may be carried out as one or more initial filtering steps.

In an example, variant sites with too low allele depth, AD, e.g. rather than just variant sites with low total coverage, may be filtered out. The inventors found that this approach reduces the number of false positives and yields more true positives.

Filtering may comprise filtering based on criteria related to mean exon coverage (MEC) of a gene, e.g. filtering out of variant sites where an associated MEC of the affected gene does not exceed a predetermined threshold.

Filtering, particularly intermediate filtering steps, may comprise filtering based on a probability that an alteration event at a variant site is likely a post-transcriptional RNA editing event. If the probability exceeds a predetermined threshold, a variant site may be filtered out.

Filtering, particularly intermediate filtering steps, may comprise filtering based on a predicted pathogenicity of an alteration event at the variant site.

Intermediate filtering steps may make use of information retrieved from data sources, for example a probability that an alteration event at a variant site is likely a post-transcriptional RNA editing event and/or predicted pathogenicity retrieved from data sources. The retrieval may make use of information comprised in the annotation.

Filtering, in particular in a downstream filtering step, may be based on a confidence associated with the prediction of a presence, absence, or undetermined presence/absence of an alteration event.

Each filtering step carried out after providing the annotation may make use of data comprised in the annotation, for example by determining whether data comprised in the annotation meets predetermined criteria.

Filtering as described above allows for ensuring accuracy, particularly in case multiple filtering steps are carried out at different stages of the method, such as when combining one or more initial filtering steps and one or more intermediate and/or downstream filtering steps.

According to the present disclosure, selecting the subset of variant sites from the plurality of variant sites may further comprise filtering the plurality of variant sites. The selecting the subset of variant sites may, for example, comprise one or more of the above-described initial filtering steps.

According to the present disclosure, the filtering may be carried out prior to selecting the subset of variant sites in accordance with the predetermined gene set. For example, the filtering may be carried out as a pre-selection step, i.e. prior to selection on the basis of the gene set. The selecting the subset of variant sites may, for example, comprise one or more of the above-described initial filtering steps.

Using filtering steps prior to and/or as part of selecting the subset of variants allows for saving resources down the line. It may also serve to catch low quality results early. Accordingly, efficiency and accuracy can be increased.

According to the present disclosure, predicting the presence and/or absence and/or an undetermined presence/absence of an alteration event at the respective variant site of the subset of variant sites may be based on coverage information of the respective variant site.

As an example, a predictive model trained on variant calling information, may be employed to predict, based on coverage information, for example comprised in or derived from variant calling information for a variant site, a level of confidence in alteration even at the respective variant site, particularly for variant sites for which no alteration event was included in the variant calling information. Additionally, for variant sites for which a level of confidence in alteration even at the respective variant site is included in the variant calling information, this level of confidence may be employed. It is noted that variant calling information does not provide such a level of confidence for variant sites for which no alteration event is comprised in the variant calling information. Accordingly, at least for these variant sites the predictive model can be employed.

Based directly or indirectly on the level of confidence, presence and/or absence and/or an undetermined presence/absence of an alteration event at the respective variant site may be determined, as is explained in more detail in other parts of the description, for example in the context of confidence scores and probabilities that can be derived therefrom.

According to the present disclosure, the predicting may further be based on the annotations of the variant sites of the subset of variant sites and/or on data derived from the annotations of the variant sites of the subset of variant sites.

For example, as explained above, the annotation may comprise information indicating a prevalence of an alteration event for a population/demographic. A high prevalence may increase confidence in determining a presence of an alteration event.

The prediction may take into account at least part of the information comprised in the annotations.

The predicting may, for example, be based on information comprised in the annotation that is not derivable from sample analysis alone. As such, it can provide context that may be valuable in combination with the sample analysis-based prediction.

Accordingly, predictions can be made more accurate by taking into account data that can give additional information not derivable from the sample analysis alone.

According to the present disclosure, the data derived from the annotations of the variant sites of the subset of variant sites may be obtained by modifying the annotations by adding further annotation data retrieved from additional data sources and/or by further filtering the subset of variant sites based on the annotations.

As already explained above, after providing an (initial) annotation making use of the predetermined dataset, e.g. the database, one or more further modification steps, i.e. steps that modify the annotation, may be carried out. A modification step may result in a modified annotation. A modified annotation may, in particular, be generated by adding further annotation data, as explained above. That is, a modification step may comprise adding annotation data. A modified annotation (including a final annotation) may be considered as being data derived from the (initial) annotation.

As also explained above, after providing the (initial) annotation, filtering of variant sites may be carried out based on the annotations. Data remaining after the filtering may also be considered data derived from the annotation.

According to the present disclosure, predicting the presence and/or absence and/or undetermined presence/absence of an alteration event at the respective variant site of the subset of variant sites may comprise determining a confidence score representative of a confidence for presence and/or absence and/or undetermined presence/absence of an alteration event at the respective variant site, in particular a false discovery rate, FDR, for an alteration event or an alteration non-event.

The confidence score may be a predicted score that indicates a level of confidence in alteration even at the respective variant site.

The confidence score may comprise a predicted confidence score that indicates a level of confidence in alteration even at the respective variant site, also referred to as pQUAL (predicted QUAL) in the detailed description.

The method comprises predicting the confidence score for an alteration event at a variant site irrespective of whether the variant calling information comprises, for the variant site, a predicted alteration event. Particularly, the method may comprise predicting the confidence score for variant sites where the variant calling information does not comprise a predicted alteration event for the variant site (i.e., absence of predicting an alteration event for the variant site in the variant calling information).

In some examples, it is determined whether the variant calling information comprises a predicted alteration event for the variant site. In case the variant calling information comprises a predicted alteration event for the variant site, the predicted confidence score may be predicted to correspond to a confidence score, also referred to as QUAL, included in the variant calling information, for example a statistic computed by a variant caller, which may for example be included in a VCF file output by the variant caller as the variant calling information. In case the variant calling information does not comprise a predicted alteration event (i.e., absence of predicting an alteration event for the variant site in the variant calling information) for the variant site, a predicted confidence score may be predicted based on the variant calling information, particularly the coverage information of the respective variant site. This may be done by means of a model trained on variant calling information.

Predicting the presence and/or absence and/or undetermined presence/absence may further comprise determining a probability of an event or non-event based on the confidence score. Probability determination depends on how the confidence score is configured/determined, i.e., how the level of confidence in an alteration event is quantified.

In some examples, the confidence score (including the predicted confidence score) may be configured to be in the format provided by variant callers, e.g. as included in the VCF file. In such cases, a logarithmic function may be used for determining the probabilities based on the following relation: $QUAL \left(or pQUAL\right) = - 10 log 10 * \left(probability p of a non-event\right)$

Probability of an event can then be determined by the relation that $p \left(event\right) + p \left(non-event\right) = 1$

Predicting the presence and/or absence and/or undetermined presence/absence may be determined based on the confidence score or the probability derived therefrom.

In particular, there may be predetermined thresholds and/or intervals associated with presence, absence, and undetermined presence/absence, respectively. Predicting the presence and/or absence and/or undetermined presence/absence may be determined by comparing the confidence score or the probability derived therefrom with the thresholds and/or intervals. For example, it may be determined whether the confidence score or probability lies within an interval associated with a presence or within an interval associated with an absence and/or within an interval associated with undetermined presence/absence of an alteration event. As another example, it may be determined whether an absolute value of the confidence score or probability exceeds a threshold associated with a non-event or a threshold associated with an event, respectively, and otherwise the result may be an undetermined presence/absence of an alteration event.

According to the present disclosure, the analysis data may further comprise the determined confidence score.

In particular, as explained herein in the context of the analysis data in general, the confidence score may be used as input for subsequent processes, such as machine learning applications.

According to the present disclosure, predicting presence and/or absence and/or undetermined presence/absence of an alteration event at the respective variant site may be based on quality of RNA sequencing data, particularly based on pre-determined sample inclusion and/or sample exclusion criteria, and/or quality of variant site, particularly determined using variant site quality scores, and/or mean exon coverage, particularly retrieved from a data source, and/or variant site depth and/or alternative allele coverage.

As will be understood from the above description, the present disclosure may entail selection, filtering and/or calculation steps taking into account these aspects. Accordingly a resulting predicted presence and/or absence and/or undetermined presence/absence can be based on one or more of said aspects.

According to the present disclosure, the method may comprise providing the analysis data as input for further automated sample analysis, particularly a machine learning, ML-based analysis, and/or for use as machine learning training data for training an ML-based sample analysis tool and/or for reporting and/or visualization for supporting a user in a technical task.

In particular, where the analysis data comprises information from the annotation, the analysis data may be used as input data for training and/or use of applications that analyze and/or learn patterns in analysis data. When it comes to training data for ML, in particular, analysis data, particularly including information from the annotation, may be used for obtaining labelled training data. Alternatively or in addition, the analysis data may be used to obtain suitably configured, e.g. structured, input for an automated sample analysis, such as ML-based analysis.

It will be understood that the above improves the capability of precision sample analysis and technical steps that are built thereon.

According to the present disclosure, the providing of the analysis data may comprise providing the annotations and/or the modified annotations and/or the final annotations and/or the predicted presence and/or absence and/or undetermined presence/absence of an alteration event at the respective variant site in the genome associated with the RNA sample.

Such analysis data can be used in the manners outlined above to allow for improved sample analysis.

According to the present disclosure, the gene set may be application-specific, and optionally may be selected taking into account information from the predetermined dataset.

Using a gene set that is application-specific allows for resource-efficiently arriving at relevant analysis data for a given application. In particular, it avoids carrying out computationally expensive steps for processing irrelevant variant sites. Apart from that, confidence of the overall process can be improved down the line, as irrelevant data for a given application could, for some further data processing, particularly machine learning, be considered noise.

According to the present disclosure, the predetermined dataset comprising the predetermined variant sites and associated annotation data may be application-specific. In particular, the predetermined data set may comprise a set of predetermined variant sites classified as relevant for a predetermined tissue type and/or medical indication and/or detectable from RNA sequencing data.

Using specific predetermined data set for a specific application (or use case) allows for the matching and retrieval of annotation data to be more efficient than when using a generalized data set, as such a generalized predetermined data set would comprise a larger number of variant sites and annotations.

Moreover, similarly to the application-specific gene set, also an application-specific predetermined data set allows for resource-efficiently arriving at relevant analysis data for a given application. In particular, it avoids carrying out computationally expensive steps for processing irrelevant variant sites. Apart from that, confidence of the overall process can be improved down the line, as irrelevant data for a given application could, for some further data processing, particularly machine learning, be considered noise.

In some examples, an application-specific dataset may be a predetermined dataset that contains only variant sites that can be consistently obtained from RNA sequencing data (e.g. variants lie on exons away from intron-exon junctions), particularly in selected tissues (e.g. KRAS is often mutated in lung but not in skin tissue), may be employed by the present method. This may ensure finding only variants that can reasonably be expected to be found, which boosts confidence.

According to the present disclosure, the method may comprise expanding the predetermined dataset in response to encountering a variant site among the subset of variant sites that has no match in the predetermined data set, the modifying comprising annotating the variant site to obtain a new predetermined variant site and associated annotation data, and adding the new predetermined variant site and the associated annotation data to the predetermined data set.

This may allow for supplementing data in the predetermined data set, for example for later use. For example, when expanding the predetermined data set as described, the data set will be supplemented in a meaningful way, i.e., with data that is likely to be useful for similar applications in the future. Specifically, if a subset of variant sites is selected, for example based on gene set, it is quite likely that a similar selection will be made in the future, e.g. for analysis using the same or similar gene set for selecting a subset of variant sites.

The present disclosure also provides use of the annotation, particularly the final annotation, obtained by a method according to the present disclosure, particularly of the method claims for analyzing an RNA sample.

The present disclosure also provides a system configured to carry out the method according to the present disclosure, particularly of the method claims.

The system comprising one or more processing devices configured to carry out the method according to the present disclosure, particularly of the method claims.

The system may optionally comprise or be connected, via data connection, to a memory device. The system may optionally comprise an output device, particularly a display device, which may be used to render a representation of the analysis data, e.g. for view by a user, e.g. for supporting the user in a technical task.

The predetermined data set may be stored on the memory device. The memory device may, for example, be a local device or a server. The data set may, for example, be stored in the form of a database. The data set may be accessed, for example, via the data connection.

The present disclosure also provides a computer program product comprising instructions which, when the program is executed by a computing system, cause the computing system to carry out the method according to the present disclosure, particularly of the method claims.

The present disclosure also provides a computer-readable medium comprising instructions which, when executed by a computing system, cause the computing system to carry out the method according to the present disclosure, particularly of the method claims.

The features and advantages outlined above in the context of the method similarly apply to the use, system, computer program product, and computer-readable medium of the present disclosure.

### BRIEF DESCRIPTION OF THE SEVERAL VIEWS OF THE DRAWINGS

In the following, embodiments of the present disclosure are described with reference to the appended figures which give background explanations and represent specific embodiments. The scope of the disclosure is however not limited to the specific features disclosed in the context of the figures, wherein
- Figs. 1a to 1c: illustrate methods for genetic variant analysis based on RNA samples according to the present disclosure;
- Fig. 2: schematically illustrates a system according to the present disclosure;
- Fig. 3: is a schematic illustration of a workflow according to the present disclosure;
- Fig. 4: illustrates the distribution of coverage depth at the KRAS G12C position across lung and skin cancer tissues for various alteration types;
- Fig. 5: is an illustration of a Rank plot of predicted G12C variant probabilities for lung cancer samples;
- Fig. 6: is an illustration of a Correlation plot of pQUAL vs QUAL scores for the KRAs G12C site in lung cancer RNA-seq samples;
- Fig. 7: illustrates the rank plot of pQUAL scores the KRAS G12C site for lung cancer RNA-seq samples;
- Fig. 8: illustrates an exemplary computing node.

### DETAILED DESCRIPTION OF EMBODIMENTS

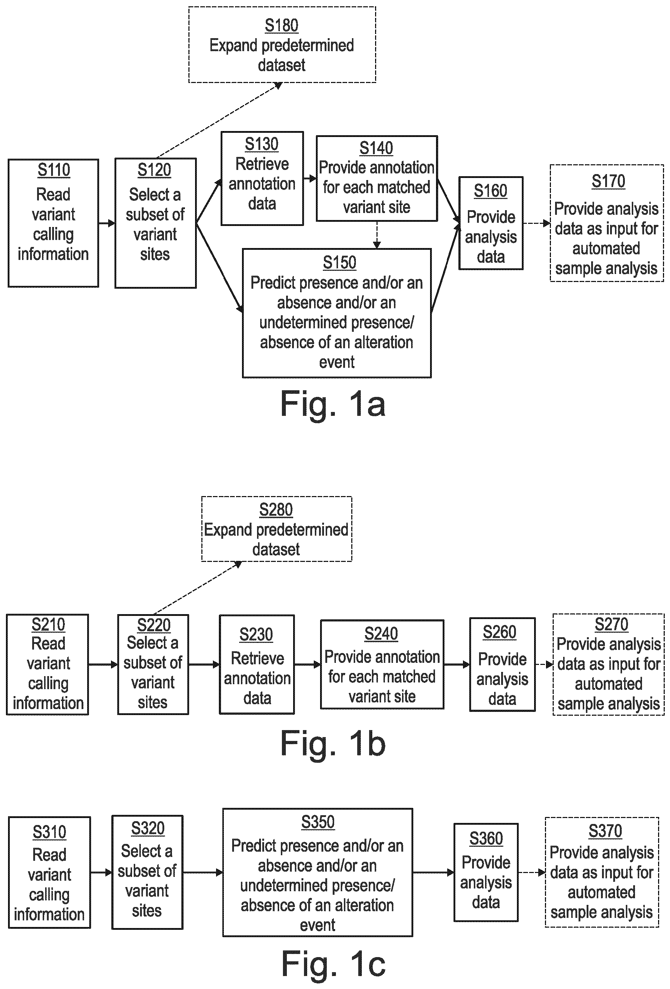

Fig. 1a illustrates the basic steps of a computer-implemented method for genetic variant analysis based on RNA samples according to the present disclosure.

The method comprises, in step S110, reading variant calling information of an RNA sample for a plurality of variant sites. The variant calling information may be obtained in preceding steps, e.g. from analyzing an RNA sample and determining variant calling information based on the RNA sequencing data associated with the RNA sample, e.g. by standard variant calling techniques.

The method comprises, in step S120, selecting a subset of the plurality of variant sites based on a predetermined gene set and independently of whether the variant calling information comprises, for the respective variant site identified by the predetermined gene set, a predicted alteration event for the variant site in the RNA sample.

The gene set may be application-specific, and optionally may be selected taking into account information from the predetermined dataset. The predetermined dataset comprising the predetermined variant sites and associated annotation data may also be application-specific, in particular, may comprise a set of predetermined variant sites classified as relevant for a predetermined tissue type and/or medical indication and/or detectable from RNA sequencing data.

Optionally, selecting the subset of variant sites from the plurality of variant sites may further comprise filtering the plurality of variant sites. The filtering may, for example, be carried out prior to selecting the subset of variant sites in accordance with the predetermined gene set.

For the subset of variant sites, steps S130, S140, and S150 are carried out.

The method comprises, for the subset of variant sites, retrieving, in step, S130, annotation data by matching at least some of the variant sites of the subset of variant sites with predetermined variant sites from a predetermined dataset, the predetermined dataset comprising annotation data associated with the predetermined variant sites.

The method comprises, for the subset of variant sites, in step S140, for each matched variant site of the subset of variant sites, an annotation comprising an identification of the variant site and corresponding annotation data.

The method comprises, for the subset of variant sites, predicting, in step S150, for one or more variant sites of the subset of variant sites, a presence and/or an absence and/or an undetermined presence/absence of an alteration event at the respective variant site in the genome associated with the RNA sample based on the variant calling information.

Predicting the presence and/or absence and/or an undetermined presence/absence of an alteration event at the respective variant site of the subset of variant sites may be based on coverage information of the respective variant site.

Optionally, the predicting may additionally be based on the annotations of the variant sites of the subset of variant sites and/or on data derived from the annotations of the variant sites of the subset of variant sites.

In some examples, predicting the presence and/or absence and/or undetermined presence/absence of an alteration event at the respective variant site of the subset of variant sites comprises determining a confidence score representative of a confidence for presence and/or absence and/or undetermined presence/absence of an alteration event at the respective variant site, in particular a false discovery rate, FDR, for an alteration event or an alteration non-event.

Predicting presence and/or absence and/or undetermined presence/absence of an alteration event at the respective variant site may based quality of RNA-sequencing data, particularly based on pre-determined sample inclusion and/or sample exclusion criteria and/or quality of variant site, particularly determined using variant site quality scores and/or mean exon coverage, particularly retrieved from a data source and/or variant site depth and/or alternative allele coverage.

The method comprises providing, in step S160, analysis data. The analysis data comprise the annotations of the variant sites of the subset of variant sites and/or data derived therefrom. Alternatively or in addition, the analysis data comprise absence and/or undetermined presence/absence of an alteration event at the respective variant site in the genome associated with the RNA sample. In case step S150 comprises determining a confidence score, the analysis data may, for example, comprise the determined confidence score.

The data derived from the annotations of the variant sites of the subset of variant sites may be obtained, for example, by modifying the annotations by adding further annotation data retrieved from additional data sources and/or by further filtering the subset of variant sites based on the annotations.

The method may optionally comprise providing, in step S170, the analysis data as input for further automated sample analysis, particularly a machine learning, ML-based analysis, and/or for use as machine learning training data for training an ML-based sample analysis tool and/or for reporting and/or visualization for supporting a user in a technical task. This may comprise providing the annotations and/or the modified annotations and/or the final annotations and/or the predicted presence and/or absence and/or undetermined presence/absence of an alteration event at the respective variant site in the genome associated with the RNA sample.

The method may optionally comprise expanding, in step S180, the predetermined dataset in response to encountering a variant site among the subset of variant sites that has no match in the predetermined data set, the modifying comprising annotating the variant site to obtain a new predetermined variant site and associated annotation data, and adding the new predetermined variant site and the associated annotation data to the predetermined data set. This may allow for expanding the predetermined data set upon encountering variant sites that have no suitable annotation data in the predetermined data set.

The method may comprise, one or more filtering steps, particularly prior to the annotating, e.g. filtering of variant sites from the plurality of variant sites based on variant site quality scores and/or mean exon coverage.

Fig. 1b illustrates another method according to the present disclosure. The method comprises steps S210, S220, S230, S240, S260, S270, and S280. For each of said steps, the description relating to steps S110, S120, S130, S140, S160, S170, and S180 apply accordingly. In step S260, of providing analysis data, the analysis data comprises the annotations of the variant sites of the subset of variant sites and/or data derived therefrom.

It is noted that in this method, a step of predicting a presence and/or an absence and/or an undetermined presence/absence of an alteration event at the respective variant site in the genome associated with the RNA sample based on the variant calling information is not shown in Fig. 1b. This serves to illustrate that such a step may be omitted. Accordingly, in this example, the analysis data may not necessarily comprise the predicted presence and/or absence and/or undetermined presence/absence of an alteration event at the respective variant site in the genome associated with the RNA sample.

Fig. 1c illustrates another method according to the present disclosure. The method comprises steps S310, S320, S350, S360, and S370. For each of said steps, the description relating to steps S110, S120, S150, S160, and S170 applies accordingly. In step S360, of providing analysis data, the analysis data comprises the predicted presence and/or absence and/or undetermined presence/absence of an alteration event at the respective variant site in the genome associated with the RNA sample.

It is noted that in this method, steps of retrieving annotation data and providing annotations are not shown in Fig. 1c. This serves to illustrate that such steps may be omitted. Accordingly, in this example, the analysis data may not necessarily comprise annotations of the variant sites of the subset of variant sites and/or data derived therefrom.

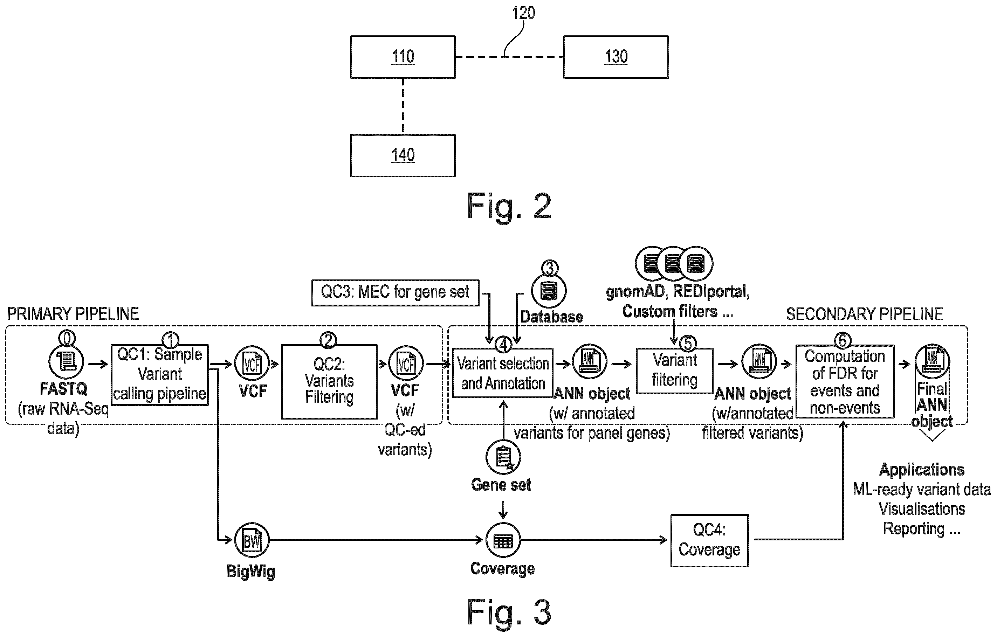

The method of Figs. 1a to 1c may be carried out by any suitable system, e.g. system according to the present disclosure, such as described in the context of Fig. 2.

Figure 2 illustrates an exemplary system 100 according to the present disclosure, the system being configured to carry out the method of the present disclosure, e.g. as described above.

In particular, the system may comprise one or more processing devices 110. The system may optionally comprise or be connected, via data connection 120, to a memory device 130. The system may optionally comprise an output device 140, particularly a display device, which may be used to render a representation of the analysis data, e.g. for review by a user.

The processing devices may be configured to carry out the method of the present disclosure. The predetermined data set may be stored on the memory device 130. The memory device may, for example, be a local device or a server. The data set may, for example, be stored in the form of a database. The data set may be accessed, for example, via data connection 120.

Further features, examples, and advantages will become apparent from the following discussion.

Many biomarkers, e.g. used in the diagnosis of disease and guidance or assignment of therapeutic intervention, are based on measurements of polymorphisms (e.g. single nucleotide, structural, or copy number variants) in DNA. For example, to be prescribed a KRAS inhibitor, the patient needs to have a particular variant in the KRAS gene. This is usually identified from the DNA in the biopsy sample.

Typically these variants are assayed using a targeted sequencing panel or a genome-wide sequencing effort that collects information on the genes of interest.

Targeted approaches are cost-efficient and offer streamlined analytical procedures but are limited in their application. On the other hand, whole exome or whole genome sequencing (WES, WGS respectively) collect information on all variants in the exome/genome, but this kind of data is more expensive to produce and analyze.

Both types of assays have the same limitation by focusing on DNA, i.e., they cannot assess the biological state of the sample. Moreover, DNA biomarkers typically just focus on simple variants in one or two genes. It is desirable to make analysis of a large number of variants cost efficient and to analyze the variants that are actually expressed. In order to do that without having to sequence both DNA and RNA, variant calling from RNA sequencing data needs to be scaled and improved to allow reliable variant calls and their interpretation.

The method of the present disclosure allows for extracting from RNA sequencing data the kinds of information that has in the past required DNA data.

The method of the present disclosure may use RNA sequencing data to identify genetic variants.

Thus from a single (e.g. total) RNA sequencing assay, the method of the present disclosure can yield biomarker calls for DNA variant-based biomarkers, in addition to yielding biomarker calls for gene expression-based (i.e. measuring gene expression levels) biomarkers.

This approach is cost effective, producing multiple kinds of information from a single analyte.

In the present disclosure, the term variant may refer to changes in the nucleotide sequence, different from the original (also called wild type, *wt*). The *original* mostly refers to the reference human sequence or reference non-human animal sequence, such as mouse, rat or monkey reference sequence. Some of these differences are inherited (i.e. germline variants), but most are acquired (i.e. somatic variants).

The method of the present disclosure does not require a targeted approach to obtain genetic variability information. For example, total RNA-Seq samples (e.g. originally collected for measuring gene expression) may be used to extract variant data. Thus, it is even possible to extract variant information out of non-target data originally obtained for a different purpose.

In a preferred embodiment the term refers to single nucleotide variants (SNV), such as single nucleotide polymorphism (SNP), and/or short INDELS. In one embodiment, the variant is not a structural variant or a copy number variant.

A variant site is a site that may contain an alteration event or not; It is a site of interest that contains an alteration event or there is a likelihood that an alteration event could occur. Accordingly, a variant site could also be predicted in the variant calling process to contain no alteration. Variant site may refer to a specific base position in a specific chromosome.

The term variant includes somatic and germline variants.

The term variant calling refers to the determination/prediction for the presence or absence of an alteration event at a variant site.

The term RNA sample refers to a biological sample, such as tissue or liquid sample. In particular, it refers to a mammalian sample, such as a sample derived from the human body (e.g. blood, salvia, tissue).

The present disclosure provides a method and system that allows to address and overcome special requirements and challenges in using RNA sequencing data compared to using DNA data, in particular including allowing for identification and interpretation of non-events.

Fig. 3 illustrates a more detailed workflow of the present disclosure that includes steps in addition to some of the steps described in Fig. 1a. The workflow may also be referred to as "analytic pipeline". The workflow can be used together with a graphical user interface that may allow a user to provide inputs. Moreover, the workflow may provide a computational interface, e.g. APIs, that may allow for providing functions and objects, such as variant tables and ANN objects. This can make information, such as the analysis data, ready and accessible for subsequent data processing, such as for ML applications.

As will be explained below, the workflow may comprise a primary analysis comprising (initial) variant calling from RNA sequencing data (i.e. determining whether there is an alteration events or not at the variant sites) and optionally filtering these variant sites based on variant quality scores, producing a VCF file of variant sites. The workflow may comprise a secondary analysis which may include additional filtering and selection steps using internal and external sources of knowledge. The workflow produces analysis data, for example, ANN objects comprising variant site (metadata) annotations and a confidence score. This analysis data can be used for downstream applications such as reporting, visualization, or machine-learning modeling.

The workflow comprises, as part of the upper branch of Fig. 3, an annotation part of the analytic pipeline. In this part of the workflow, a predetermined dataset is employed that comprises annotation data associated with predetermined variant sites. In the example shown in Fig. 3, this predetermined data set is shown in the form of a database. It may be considered a database or atlas of variants and their respective description (examples of which are given below).

Using such a dataset, e.g. database, improves efficiency of the workflow. In particular, efficiency of variant annotation can be increased because identified variants in a sample can now simply be matched to database entries instead of having to *de novo* annotate each variant, potentially using multiple tools and sources of external knowledge. The annotation data associated with a variant site may be considered to be metadata of said variant. In the dataset, annotation data originating from different sources may be included. The dataset may be application-specific. For example, the dataset may only include the variants that can be reliably called from RNA sequencing data in specific tissues and associated annotation data. As an example, the dataset may include variants (and associated annotation data) from a target panel of genes important in a particular disease indication (which is an example of a gene set as specified in the method of the present disclosure).

Furthermore, in the second (lower) branch of Fig. 3, the workflow may encompass predicting, for one or more variant sites, a presence and/or an absence and/or an undetermined presence/absence of an alteration event at the respective variant site in the genome (i.e. at DNA level) associated with the RNA sample based on the variant calling information. A confidence for event and/or for non-events may be provided, for example in the form of a confidence score. In the workflow shown in Figure 3, this involves computing a false discovery rate, FDR, for events and non-events. An undetermined presence/absence (event/non-event) may also be the result of this computation/prediction step.

The prediction may entail, for example, computing a confidence value (p-value or false discovery rate) for a particular variant site which indicates the confidence of the presence of an alteration at the variant site (if it was found to be present in the variant calling data) or confidence in the absence of an alteration at the variant site (if it was not found in the variant calling data).

As part of the workflow, a quality control procedure may be implemented, which may include controlling one or more of the following quality measures: quality of sequencing data, quality of called variant, mean exon coverage (MEC), and variant coverage information.

The workflow or analytical pipeline may include a primary analysis portion, indicated by the numbers 0, 1, and 2 in Fig. 3. These may be steps the output of which is used by the method of the present disclosure or at least in part steps that are part of the method of the present disclosure. However, for the steps of the primary analysis portion, known technical solutions and implementations can be employed.

**[0]** In an optional first step, sample data may undergo quality control (QC1) to verify if the samples meet inclusion criteria for data analysis, e.g. inclusion criteria set for both variant calling and gene expression analysis of the data. The filter may be provided in the form of a Python script. Exemplary filters may comprise:
- number of reads; there needs to be at least 30 million reads (or pairs of reads) per sample after trimming (where trimming may refer to removing sequencing artifacts (adapters) and low-quality sequencing bases, which can result in some sequencing reads to be reduced to lengths below threshold (possibly even zero), effectively removing the sequencing reads from the analysis)
- assigned reads; warning if <10 million reads are assigned, fail if <1 million reads are assigned
- GC content (guanine-cytosine content); if GC content is above 60%, a warning is issued, the sample fails with GC content >80
- duplication rate; warning if >75%, sample fails if >90% duplication rate.
- rRNA; warning if rRNA content >10%, fail if >60%
- globin: warning if globin RNA content >3%, fail if >5

**[1]** A step that can yield variant calling data and, hence, can be referred to as variant calling step, that determines in input RNA sequencing data (for example in a FASTQ file or other, e.g. text-based, file for storing biological sequence ) variant sites (for example in a VCF file or other, e.g. text, file for storing gene sequence or DNA sequence variants). This may be done using a reference of exonic region, a list of known mutations (e.g. from a dbSNP file, e.g. retrieved from a database of short genetic variations), and optionally other inputs. The step can follow the GATK's best practices (i.e. mapping to reference using e.g. STAR, Data cleanup using e.g. MergeBamAlignment MarkDuplicates, Reformating using e.g. SplitNCigarReads, Base Quality Recalibration using e.g. BaseRecalibrator, ApplyRecalibration, AnalyzeCovariates, Variant Calling e.g. using HaplotypeCaller, Variant Filtering using e.g. VariantFiltration; for details see https://gatk.broadinstitute.org/hc/en-us/articles/360035531192-RNAseq-short-variant-discovery-SNPs-Indels).

In principle, known tools, steps, interactions, and settings can be used. An implementation can, for example, be the system Genialis^{™} Expressions.

**[2]** In a subsequent step (labeled "QC2"), variants in the VCF file are first filtered by quality. Known filtering could, in theory, be employed. In this example, however, variant sites with too low allele depth, AD, are filtered out (rather than conventional filtering out variant sites with low total coverage). The inventors found that this approach reduces the number of false positives and yields more true positives.

In the present example, variant sites are removed if
- QD < 2; confidence of the call, QD being a variant confidence normalized by unfiltered depth of variant samples (QD) (e.g. QUAL/DP)
- FS > 30; Fisher strand, i.e. strand bias at the site, with larger values meaning larger bias
- AD < 5; alternative allele depth, i.e. the variant needs to be supported with at least 5 reads

Steps [1-2] may be implemented and provided, for example, by Genialis^{™} Expressions.

Common to these steps is that each sample is treated independently (e.g. regardless of the patient cohort) and completely (i.e. all genes, genome-wide, are considered, all variants that meet quality criteria are included).

The resulting VCF file may be a text file. However, this file often requires further processing before its information can be meaningfully employed.

This further processing can be done in what will be referred to as "secondary analysis" and "selection of variants" below. The secondary analysis may, for example, also yield tabular data for selected genes or variants.

The secondary analysis may include some or all of the aspects [3] to [6] outlined below.

Aspects [3] to [5] relate to providing of annotations as described above and in the claims.

**[3]** Illustrates that a dataset comprising annotation data, such as a database (DB), of variant annotation data is employed in the present disclosure, i.e., the predetermined dataset of the claims. The method of the present disclosure optionally entails the creation of such a dataset.

For the creation of such a dataset, information from multiple sources can be pulled. As an example clinical annotations are collected from a database of clinical significance of variants, for example the ClinVar database (which archives and aggregates information about relationships among variation and human health), and additional annotations can, for example, be derived from genetic variant annotation tools, such as the tool SnpEff.

Using the dataset comprising annotation data is a significant procedural improvement over the known procedures, in which individual VCFs are annotated (e.g. with a tool such as SnpEff) separately each time an analysis is carried out. Instead, in the present disclosure the variant annotation data may be created only once and kept in the dataset, e.g. a database.

The dataset, as also explained above, may be application-specific. For example, a dataset, e.g. database, that contains only variants that can be consistently obtained from RNA sequencing data (e.g. variants lie on exons away from intron-exon junctions) in selected tissues (e.g. KRAS is often mutated in lung but not in skin tissue) may be employed by the present method. This may be employed to ensure finding only variants that can reasonably be expected to be found, which boosts confidence.

Data for the dataset, e.g. the data set, may be compiled from multiple sources, as explained above. There are academic papers showing that no one source (e.g. the database ClinVar) gives a complete overview of the clinically relevant variants, their presence in different populations, and documented or speculated clinical impact, so a compilation can improve overall accuracy.

The predetermined dataset may comprise, for each predetermined variant site, a variantlD, a variant position in the reference genome, an impact of an alteration at a variant site, an effect (pathogenicity) of an alteration at a variant site, an amino acid change, presence and/or frequency of an alteration at a variant site in population/demographic, other clinical effect metadata.

The database, as mentioned above, can be application (task) specific, e.g., for lung cancer. This may allow for ensuring accuracy and also efficiency in retrieving relevant annotation data in the workflow.

Use of a dataset, e.g. database, with annotation data allows for simply matching individual variants with entries in the dataset, e.g. database entries. This allows highly efficient annotation, e.g. compared to the known approach that (newly) annotates the variants in every sample every time.

**[4]** Indicated by number 4 is a predetermined gene set which identifies selected genes (also called "panel genes"). Thus, for example, only variant sites located on selected genes are selected to become part of the subset of variant sites of the present disclosure. The selected variant sites may be further filtered by class and putative impact (see below). For the subset of variant sites, annotation data may be retrieved from the dataset, e.g. database, annotations may be generated, and the variant sites may be reported together with their annotations, which in turn are derived from the dataset, e.g. database indicated and explained in [3].

A step labeled QC3 may also be carried out, which may be considered as a selection or filtering step. The step may select only genes with minimum mean exon coverage (MEC), for example MEC >10, reads (the minimum MEC indicating data suitable for downstream analysis). In other words, the variants on genes not exceeding this threshold are filtered out. Selection based on MEC could optionally also be part of QC2.

As indicated in the flowchart, after variant selection and annotation data retrieval, an annotation may be generated and optionally be output.

A set of data, referred to herein as ANN object, may be generated. In this example, the ANN object contains information on variant sites, for example for all samples in a cohort. The ANN object may comprise information for one or more of the following variables: "CHROM", "POS", "ID", "QUAL", "REF", "ALT", "DP", "FILTER", "EFF", "Allele", "Description", "Annotation_Impact", "Gene_Name", "Feature_ID", "HGVS.p". Additionally, the ANN object may comprise sample metadata. Optionally, functions may be provided that can provide consistency of nomenclature, such as translating three-letter aminoacid designations into single-letter designations, for example, changing p.Gly12Cis to the canonical G12C. The variables mentioned above may represent the following information:
- **CHROM:** Chromosome - the chromosome name or number where the variant is located.
- **POS:** Position - the 1-based position of the variant site on the chromosome.
- **ID:** Identifier - a unique identifier for the variant site, often corresponding to a database entry such as dbSNP ID.
- **QUAL:** Quality - a Phred-scaled score related to a probability that the variant call is correct, with higher values indicating higher confidence (cf. Eq. 1).
- **REF:** Reference Allele - the reference base(s) at this position in the genome.
- **ALT:** Alternate Allele - the alternate base(s) observed at this position instead of the reference allele.
- **DP:** Depth - the total depth of reads covering the variant site, indicating how many reads support this variant site.
- **FILTER:** Filter status - indicates whether the variant passed specific filtering criteria. Common values include PASS (variant passed all filters) or names of filters it failed.
- **EFF:** Predicted Variant Effect - a field typically containing a summary of variant effect annotations generated by a tool like SnpEff.
   Within the **EFF** field, specific annotations can be broken down further, such as:
   ▪ **Allele:** The specific alternate allele being annotated.
   ▪ **Description:** A general description of the predicted variant effect, such as "missense_variant" or "synonymous_variant."
   ▪ **Annotation_Impact:** The impact level of the variant, such as "HIGH," "MODERATE," "LOW," or "MODIFIER," based on its likely effect on gene function.
   ▪ **Gene_Name:** The name of the gene affected by the variant, e.g. according to the HGNC (HUGO Gene Nomenclature Committee) or another gene database.
   ▪ **Feature_ID:** The ID of the specific transcript, coding sequence, or gene feature affected by the variant.
   ▪ **HGVS.p:** Human Genome Variation Society (HGVS) protein notation - describes the predicted protein-level change caused by the variant, typically starting with "p." followed by the amino acid change, such as p.Val600Glu.

[5] Optionally, as indicated in number [5], selected variant sites may be further annotated and/or filtered using additional external information. The following is a non-exhaustive list of such resources:
1. Use of additional annotation options from SnpEff:
   a. Cancer tissue analysis: Somatic vs Germline mutations can be calculated. This is very useful for oncology research.
   b. Loss of Function (LOF) assessment: SnpEff can estimate if a variant is deemed to have a loss of function on the protein.
   c. Nonsense mediated decay (NMD) assessment: Some mutations may cause mRNA to be degraded thus not translated into a protein. NMD analysis marks mutations that are estimated to trigger nonsense mediated decay.
2. An RNA editing database, such as REDIportal database, can be used to determine if RNA variants resulted from post-transcriptional RNA editing events rather than changes in the DNA, and these variants can be removed from further study. This may be considered a filtering step.
3. A database containing information on pathogenicity of missense variants, such as AlphaMissense, can be used to predict pathogenicity of variants that have not been experimentally/clinically confirmed as pathogenic.
4. Custom annotation databases can be built to annotate in detail high-impact variants in selected genes, e.g. collated from research.
5. Clinical metadata of samples may be provided together with variant calling information, such as pulled from Genialis^{™} Expressions.
6. Population frequencies, for example, prevalence in certain populations.

**[6]** Indicated in number [6] is providing coverage information, e.g. to the ANN object, for at least some variant sites is added. The coverage information may, for example, be part of or computed from variant calling information. Coverage information may be computed from variant calling information provided in a data format configured for efficient processing of condensed, optionally continuous, data, e.g. data that will be displayed in a Genome Browser as a graph. For example, coverage information may be computed from variant calling information provided in an indexed binary format, e.g. BigWig.

A presence and/or an absence and/or an undetermined presence/absence of an alteration event at the respective variant site in the genome associated with the RNA sample based on the variant calling information, particularly based on coverage information and (other) metadata. In particular, a confidence score may be computed.

An output of analysis data, for example in a final ANN object, may be provided, for example for downstream applications, which will be described further below.

The analysis data, e.g. the ANN object, can comprise at least some of the following:
Information on variants on all selected variant sites (subset of variant sites) in selected genes, in particular together with known or predicted pathogenicity and other metadata, total and AD coverage at these sites, custom annotations of selected variant sites, and clinical information about the subjects of study (e.g. patients). This allows for a comprehensive interpretation of the variant site.

At any point during the workflow, information in the ANN object may be used for filtering, depending on the application at hand.

For example, filtering may have to go beyond the "pathogenic"/"likely-pathogenic" annotation that can be provided, e.g. by ClinVar, to account for variants not recorded, e.g. in ClinVar.

Filters may comprise one or more of the following:
o Putative_impact, a parameter computed by the tool SnpEff; variants with the following putative_impact classes are retained: {HIGH, MODERATE, MODIFIER}, class {LOW} is filtered out.
o Variants with the following annotation classes (e.g. given by the tool SnpEff: "A program for annotating and predicting the effects of single nucleotide polymorphisms, SnpEff: SNPs in the genome of Drosophila melanogaster strain w1118; iso-2; iso-3.", Cingolani P, Platts A, Wang le L, Coon M, Nguyen T, Wang L, Land SJ, Lu X, Ruden DM. Fly (Austin). 2012 Apr-Jun;6(2):80-92. PMID: 22728672) are retained: {inframe_insertion, disruptive_inframe_insertion, inframe_deletion, disruptive_inframe_deletion, frameshift_variant, missense_variant, rare_amino_acid_variant, splice_acceptor_variant, splice_donor_variant, stop_lost, start_lost, stop_gained}

The workflow in Fig. 3 indicates potential applications downstream of the above steps. Non-limiting examples for such applications are provided below:
- Example 1: Reporting and visualization. Variant sites can be reported in a form of tables and plots.
- Example 2: Sample filtering. Filtering may be carried out on the basis of annotations, e.g. finding only samples with predicted pathogenic variant in a target gene. Filtering can also be based on the presence or absence of a alteration (e.g. based on the confidence score). For example, a filter may allow for selecting only samples without a alteration in a certain position. In this case samples with the alteration and samples with a low probability of non-event (i.e., samples where presence or an undetermined presence/absence of an alteration event are determined) are filtered out.
- Example 3: Machine learning applications. The output of the process may be ML-ready data, e.g. variants in rows, samples in columns, in a ML-ready matrix format. This can be used in any number of modelling applications.

Some examples use cases for the method of the present disclosure, for example the workflow as described above, will be described in the following:
For example, results similar to a variant panel can be reported for oncology applications. As another example, detectable substitutions (SNPs) and short insertions/deletions (INDELs) in exonic regions of genes involved in homologous recombination repair (HRM), mismatch repair (MMR) and replication stress processes can be reported, and these could be features for modeling DNA damage repair biology.

Furthermore, these are some ways in which, according to the present disclosure, variants may be reported and visualized:
- A dataset and patient specific heatmap containing filtered variant information with accompanying information on allele frequency in population, pathogenicity, type of variant and alteration present can be plotted along with QC flags of the geneset and samples
- A patient specific report can be generated in a table style format for likely pathogenic/pathogenic variants or variants that are of unknown significance
- Interpretation of events/non-events, e.g., event/non-event confidence score
- Per patient per gene figures

In the above-described example, the primary analysis pipeline may be implemented on Genialis Expressions, for example. Filters, class, and functions for the ANN object may be implemented as utility functions in ReSDK.

### Predicting presence and/or absence and/or undetermined presence/absence of an alteration event

In the following, an example for predicting (e.g. steps S150 or S350), for one or more variant sites of the subset of variant sites, a presence and/or an absence and/or an undetermined presence/absence of an alteration event at the respective variant site in the genome associated with the RNA sample based on the variant calling information will be provided.

In the following example, the presence and absence of an alteration event will be referred to as event and non-event respectively.

The method in this example estimates the probability of an event (alteration event, also referred to below as variant), and the probability of an non-event (the absence of an alteration event, also referred to below as non-variant).

As an example, the method may leverage information from the variant calling information, e.g. the QUAL score in the VCF file (as described in detail above), using the following relationship: $QUAL = - 10 log 10 * \left(probability that the site has no variant\right)$

The QUAL score is a statistic computed by the variant caller (primary analysis). More specifically, the QUAL score is a Phred-scaled quality score indicating a variant (e.g. variant specified in the ALT field of the VCF file).

For example, if ALT indicates that there is variant, QUAL indicates confidence in the variant (which is linked to the probability of no alteration event as per Eq. 1).

The probability of event and non-event, for a variant-site, are related with the following equation: $p \left(event\right) + p \left(non-event\right) = 1$

The method of the present disclosure, contrary to known methods, entails analysis of two cases.

In case 1 a variant is present according to/in the VCF file of the RNA sequencing sample. The non-event probability is determined from the corresponding QUAL score in the VCF file and the event probability is computed by subtracting the non-event probability from 1.

In case 2, if the variant is not present in the VCF file, a non-event probability is determined using a machine learning algorithm. For example, a linear model is fitted on QUAL scores of all detected variants in the VCF file using as features (1) the total number of reads at the site of interest and (2) the proportion of reads supporting the variant. The target variable that is predicted with this model is a pQUAL (predicted-QUAL) score for the site that is not in the VCF file, and gives confidence for event at this site that is related to (non)event probability according to Eq. 1 and 2.

The number of reads may also be referred to as coverage. For example, using the actual total number of reads at the site of interest and a chosen predetermined minimum proportion of reads supporting the variant, the fitted linear model predicts a corresponding confidence score (pQUAL) representative of a **non-event** probability. The choice of the predetermined minimum proportion of reads supporting the variant may, for example, depend on the desired tradeoff between sensitivity and specificity. A higher sequencing depth allows for a lower threshold for the proportion of reads supporting the variant, leading to a higher number of detected variants.

The predicted probability of a non-event at a site not included in the VCF may be related with the confidence score (pQUAL) as follows: $pQUAL = - 10 log_ 10 * \left(predicted probability of a non-event\right)$

The **non-event** probability may be determined from the pQUAL score and the event probability can be computed by subtracting the non-event probability from 1 (Eq. 2).

The method may further entail p-value adjustment for multiple hypothesis testing. For example, all non-event probabilities may be adjusted for multiple hypothesis testing, e.g. using the Benjamini-Hochberg procedure. The number of hypotheses tested in this example is equal to the number of events that are tested for multiplied by the number of RNA sequencing samples. The adjusted non-event probabilities can be computed with a chosen/predetermined false discovery rate (FDR). The corresponding event probabilities may then be recomputed by subtracting the non-event probabilities from 1.

In an example, pQUAL scores and/or event and/or non-event probabilities may be determined for every variant site of the subset, independently of whether the variant calling information comprises a predicted alteration event for the variant site in the RNA sample.

Known variant calling software produces a QUAL score (only) for all positions where it identified a variant (i.e., where it identified an alteration event). E.g., only if a variant is identified in the VCF file, there will be a corresponding QUAL score. But not all positions where a variant was not identified (i.e., where variant calling information implies a non-event) are reliable. Present techniques do not allow for handling (potential) non-events accurately. For example, contrary to the method of the present disclosure, in known methods there is no score that could distinguish this position.

The present disclosure allows for handling non-events accurately, for example by computing a confidence score pQUAL for events, as explained above.

Accordingly, with the approach of the present disclosure, it is possible to accurately distinguish between likely true events, likely true non-events and cases where presence or absence of an event remains undetermined.

It is noted that the determination of the confidence score pQUAL above did not rely on information from the annotation, e.g. data retrieved from the database comprising annotation data. However, it is possible to leverage such information from the annotation, e.g. data retrieved from the database, when determining the confidence score. Two examples are provided below, but the present disclosure is not limited to said examples:
- Example 1: Population frequencies. Some databases, e.g. genome aggregation databases such as GnomAD, provide information on the presence of mutations in different demographics. If, for example, a mutation is present in high frequency in Asian populations but is virtually absent in black people, and if a sample in question comes from a person of Asian descent, such a mutation could be evaluated as more likely. A similar example could be for tissues of origin. For example, the KRAS gene is expressed in skin to a similar degree as in lung, but it is very rarely mutated in skin (<1%) compared to lung (>25%). Information like this can be captured in the database comprising the annotation data. Such information can be taken into account when determining the confidence score.
- Example 2: Hotspots of mutations. Some regions of the genome are known to be more susceptible to variation than others. Information reflecting hotspots may be comprised in the database comprising annotation data, included in the annotation, and taken into account for determining the confidence score.

### Experimental Section

A description of experiments done in support of the technical effects of the method and system of the present disclosure are outlined below.

In particular, a method as shown in the flowchart shown in Fig. 3, was followed, referred to herein as RNA-based genetic variant analysis. Positive and negative control samples for KRAS G12C mutation are selected from lung and skin cancer cell lines in the public CCLE database. This variant is called in all samples and the calls are compared to the ground truth from the database. Confidence scores are computed and interpreted.

### Data

Cancer Cell Line Encyclopedia (CCLE) lung and skin cancer RNA-seq samples were used in this experiment. In total, 168 lung cancer and 45 skin cancer samples were used. This dataset provided a diverse representation of cancer subtypes relevant to the analysis of the KRAS G12C variant, a key mutation of interest in lung cancer research.

### Gene and variant information

The gene of interest, KRAS (Kirsten Rat Sarcoma Viral Oncogene Homolog), is a well-known oncogene involved in cell signaling pathways that regulate cell growth and survival. KRAS mutations, particularly in codon 12, play a critical role in several types of cancer, including lung and colorectal cancer. The G12C variant (glycine to cysteine mutation at position 12) is particularly relevant in non-small cell lung cancer (NSCLC), where it has been implicated in tumorigenesis and serves as a therapeutic target. Given its importance, this study focuses on accurately detecting the KRAS G12C variant in both lung and skin. While the gene is expressed in both types of cell lines, it is rarely mutated in skin cancer cell lines, thus skin cancer cell line samples represent a negative control.

### Pipeline information

The variant calling pipeline along with the method to produce confidence scores are explained in detail above in the context of Fig. 3.

### Results and Discussion

These experiments demonstrated the capacity of the RNA-seq variant calling pipeline to detect the KRAS G12C variant within lung and skin cancer cell lines of the Cancer Cell Line Encyclopedia (CCLE) dataset. The experiment highlighted key findings for the G12C variant detection in terms of coverage depth, distribution across tissue types, and reliability of RNA variant calls compared to DNA-based reference calls (ground truth reported in the database). Moreover, they test the performance of the confidence scores and their ability to interpret different types of non-events.

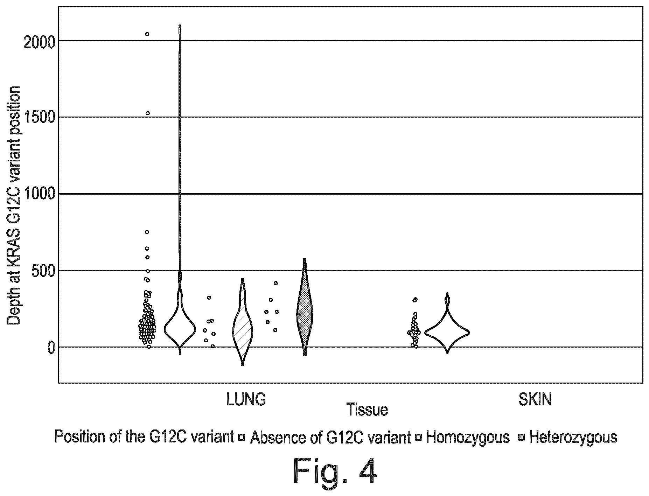

Figure 4 illustrates the distribution of coverage depth at the KRAS G12C position across lung and skin cancer tissues for various alteration types (wild-type, homozygous, and heterozygous). This violin plot provides a kernel density estimate (KDE) to visualize depth variability within each group. Lung cancer samples, especially those with heterozygous G12C, generally show higher coverage depths, reinforcing the robust detectability of this variant in lung tissues. The variation across alteration types supports using RNA-seq data to identify specific variants with greater sensitivity within lung tissue samples. Generally, skin samples did not include any KRAS G12C variants, while coverage for detection is sufficient. In contrast, skin tissue samples in general exhibited lower coverage. Note: this kind of information may be used in determining which genes and which mutations should be included in our database of variants for any particular disease indication.

Fig. 4 is a Violin plot displaying the depth of coverage at the KRAS G12C variant position across lung cancer tissue (N = 168) and skin cancer tissue (N = 45) types and alteration types (absence of G12C variant, homozygous, heterozygous). The depth distribution for each tissue and alteration type is shown, with kernel density estimates (KDE) used to depict the distribution of the data. While the expression of the KRAS gene, approximated by the sequencing depth in RNA-Seq data, is similar in lung and skin, the skin samples were not mutated in the KRAS gene.

The accuracy of the RNA variant calling pipeline was evaluated against DNA variant calls, as shown in Table 1, which presents a confusion matrix comparing predicted RNA-seq variant calls to DNA-based reference calls. The pipeline correctly identified 12 samples harboring the G12C mutation and 200 cases with the absence of the G12C variant. The high accuracy demonstrated by the RNA-seq pipeline, reflected in the low false positive rate, emphasizes its reliability in detecting the KRAS G12C variant, supporting its suitability for clinical applications in variant calling. Furthermore, the confidence scores supporting the accuracy of the pipeline are outlined in the next section.

**Table 1: Confusion matrix.**

| | | |
|---|---|---|
| Absence of G12C variant (Actual) | 200 | 1 |
| G12C (Actual) | 0 | 12 |
| | Absence of G12C variant (Predicted) | G12C (Predicted) |

Table 1 is a Confusion matrix showing the comparative RNA vs DNA variant calling results for identifying presence of absence of KRAS G12C variant. The RNA variant calling pipeline correctly identified 12 G12C samples and 200 samples where it is not present.

The ground truth information (DNA-Seq variant calls reported in the CCLE database) for the presence of the G12C variant, first introduced in Table 1, is summarized below in Table 2. The table outlines the number of cases with the G12C variant and without the G12C variant in lung and skin cancer samples from the CCLE dataset. All 12 samples with the G12C variant are found exclusively in lung cancer samples, with none detected in skin cancer samples. This tissue-specific distribution aligns with the known prevalence of the KRAS G12C mutation in lung cancers and its rarity in skin cancers.

**Table 2: Number of samples with ground truth annotated for lung and skin samples in the CCLE dataset.**

| | **Lung cancer samples** | **Skin cancer samples** | **Total** |
|---|---|---|---|
| G12C variant | 12 | 0 | 12 |
| Without G12C variant | 156 | 45 | 201 |
| Total | 168 | 45 | 213 |

### Confidence scores

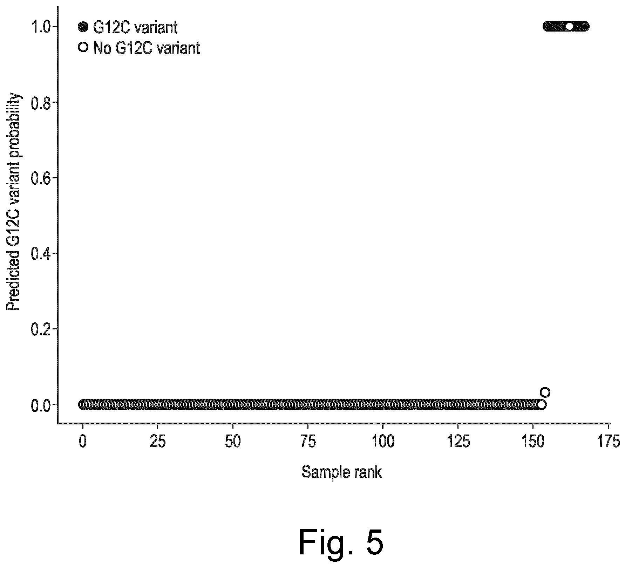

These are computed for the G12C variant site for all 213 samples using the method from the disclosure document. The G12C variant probabilities, computed from the pQUAL scores, are then plotted on a rank plot for lung cancer samples (Figure 5). The model is able to differentiate between G12C-positive and negative samples. All but one sample without the variant (gray points) are correctly predicted with probabilities near zero, while the G12C-positive samples (blue points) are all correctly predicted as mutated with probabilities close to one, showing excellent separation.

Figure 5 is a Rank plot of predicted G12C variant probabilities for lung cancer samples. Samples without the G12C variant are marked in gray, and G12C-positive cases are marked in blue. The model shows clear separation between the presence/absence G12C variant in lung cancer samples with a misclassification explainable by low coverage at the variant site and limited sample size for FDR corrections.

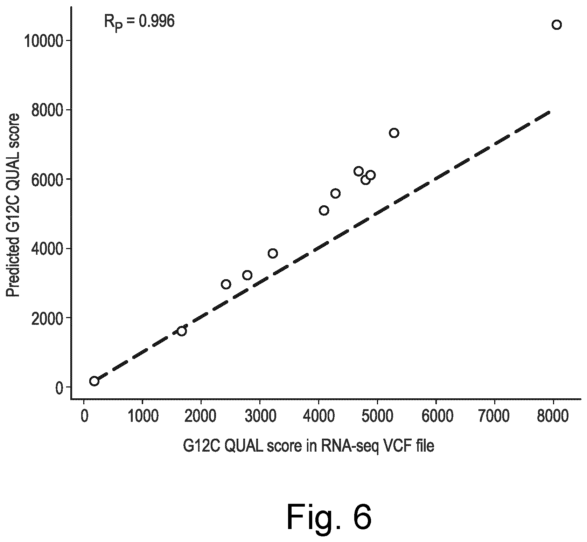

Figure 6 presents a correlation plot comparing pQUAL scores and true QUAL scores from RNA-seq VCF files for lung cancer samples with identified variants at the site G12C. The strong correlation confirms the model's effectiveness in predicting QUAL score distributions for lung cancer RNA-seq data.

Figure 6 is a correlation plot of predicted vs. true G12C QUAL scores in lung cancer RNA-seq samples. Pearson's correlation coefficient of 0.996 confirms the high performance of the predictive model of variant QUAL scores. The dashed diagonal line represents perfect correlation.

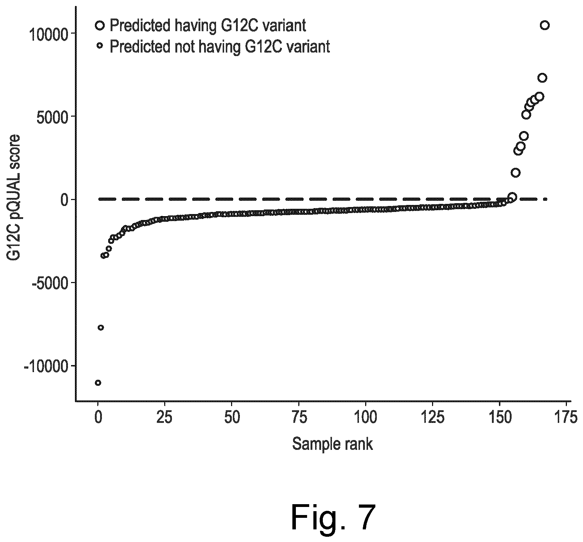

Figure 7 illustrates the rank plot of predicted G12C QUAL scores for lung cancer RNA-seq samples, providing insight into the model's classification. Samples predicted as G12C-positive (bigger circles) generally exhibit high positive QUAL scores, whereas G12C-negative samples (smaller circles) mostly cluster around or below zero QUAL scores with some outliers on both ends. This pattern underscores the model's strong capacity to separate samples based on the G12C variant presence.

Figure 7. A rank plot of predicted G12C QUAL scores in lung cancer RNA-seq samples. The plot shows pQUAL scores ranked by sample, where G12C-positive samples are marked as bigger gray and G12C-negative samples in smaller gray circles. The dashed horizontal line at zero serves as a threshold, separating predictions for samples classified as likely having the G12C variant (positive QUAL scores) from those predicted as not having the G12C variant (negative QUAL scores). The figure demonstrates the model's ability to effectively differentiate between the two classes, with most samples aligning appropriately to their predicted categories.

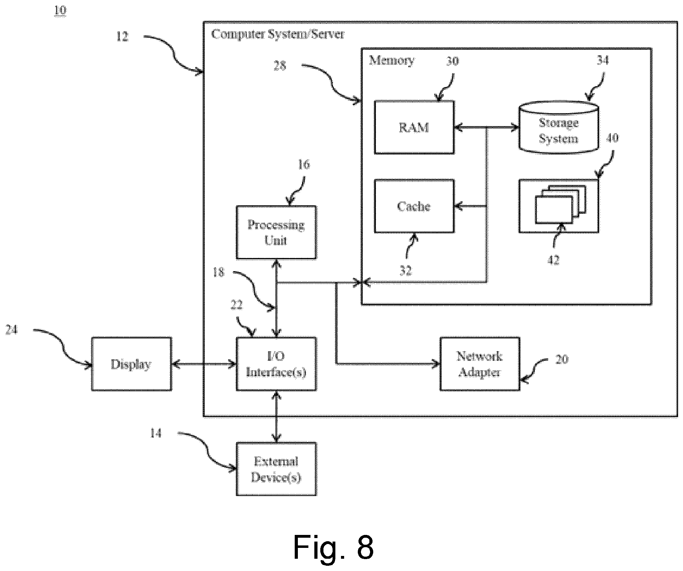

Referring now to Fig. 8, a schematic of an example of a computing node is shown. Computing node 10 is only one example of a suitable computing node and is not intended to suggest any limitation as to the scope of use or functionality of embodiments described herein. Regardless, computing node 10 is capable of being implemented and/or performing any of the functionality set forth hereinabove.

In computing node 10 there is a computer system/server 12, which is operational with numerous other general purpose or special purpose computing system environments or configurations. Examples of well-known computing systems, environments, and/or configurations that may be suitable for use with computer system/server 12 include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, and distributed cloud computing environments that include any of the above systems or devices, and the like.

Computer system/server 12 may be described in the general context of computer system-executable instructions, such as program modules, being executed by a computer system. Generally, program modules may include routines, programs, objects, components, logic, data structures, and so on that perform particular tasks or implement particular abstract data types. Computer system/server 12 may be practiced in distributed cloud computing environments where tasks are performed by remote processing devices that are linked through a communications network. In a distributed cloud computing environment, program modules may be located in both local and remote computer system storage media including memory storage devices.

As shown in Fig. 8, computer system/server 12 in computing node 10 is shown in the form of a general-purpose computing device. The components of computer system/server 12 may include, but are not limited to, one or more processors or processing units 16, a system memory 28, and a bus 18 that couples various system components including system memory 28 to processor 16.

Bus 18 represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, Peripheral Component Interconnect (PCI) bus, Peripheral Component Interconnect Express (PCIe), and Advanced Microcontroller Bus Architecture (AMBA).

Computer system/server 12 typically includes a variety of computer system readable media. Such media may be any available media that is accessible by computer system/server 12, and it includes both volatile and non-volatile media, removable and non-removable media.

System memory 28 can include computer system readable media in the form of volatile memory, such as random access memory (RAM) 30 and/or cache memory 32. Computer system/server 12 may further include other removable/non-removable, volatile/non-volatile computer system storage media. By way of example only, storage system 34 can be provided for reading from and writing to a non-removable, non-volatile magnetic media (not shown and typically called a "hard drive"). Although not shown, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to bus 18 by one or more data media interfaces. As will be further depicted and described below, memory 28 may include at least one program product having a set (e.g., at least one) of program modules that are configured to carry out the functions of embodiments of the disclosure.

Program/utility 40, having a set (at least one) of program modules 42, may be stored in memory 28 by way of example, and not limitation, as well as an operating system, one or more application programs, other program modules, and program data. Each of the operating system, one or more application programs, other program modules, and program data or some combination thereof, may include an implementation of a networking environment. Program modules 42 generally carry out the functions and/or methodologies of embodiments as described herein.

Computer system/server 12 may also communicate with one or more external devices 14 such as a keyboard, a pointing device, a display 24, etc.; one or more devices that enable a user to interact with computer system/server 12; and/or any devices (e.g., network card, modem, etc.) that enable computer system/server 12 to communicate with one or more other computing devices. Such communication can occur via Input/Output (I/O) interfaces 22. Still yet, computer system/server 12 can communicate with one or more networks such as a local area network (LAN), a general wide area network (WAN), and/or a public network (e.g., the Internet) via network adapter 20. As depicted, network adapter 20 communicates with the other components of computer system/server 12 via bus 18. It should be understood that although not shown, other hardware and/or software components could be used in conjunction with computer system/server 12. Examples, include, but are not limited to: microcode, device drivers, redundant processing units, external disk drive arrays, RAID systems, tape drives, and data archival storage systems, etc.

The present disclosure may be embodied as a system, a method, and/or a computer program product. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

While embodiments of the present disclosure have been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

## Claims

1. A computer-implemented method for genetic variant analysis based on RNA samples, the method comprising:
reading (S110, S210, S310) variant calling information of an RNA sample for a plurality of variant sites;
selecting (S120, S220, S320) a subset of the plurality of variant sites based on a predetermined gene set and independently of whether the variant calling information comprises, for the respective variant site identified by the predetermined gene set, a predicted alteration event for the variant site in the RNA sample;
for the subset of the plurality of variant sites:
retrieving (S130, S230) annotation data by matching at least some of the variant sites of the subset of variant sites with predetermined variant sites from a predetermined dataset, the predetermined dataset comprising annotation data associated with the predetermined variant sites,
providing (S140, S240), for each matched variant site of the subset of variant sites, an annotation comprising an identification of the variant site and corresponding annotation data, and
predicting (S150, S350), for one or more variant sites of the subset of variant sites, a presence and/or an absence and/or an undetermined presence/absence of an alteration event at the respective variant site in the genome associated with the RNA sample based on the variant calling information; and
providing (S160, S260, S360) analysis data comprising the annotations of the variant sites of the subset of variant sites and/or data derived therefrom and the predicted presence and/or absence and/or undetermined presence/absence of an alteration event at the respective variant site in the genome associated with the RNA sample.

2. The method of claim 1, wherein the method comprises
filtering of variant sites from the plurality of variant sites based on variant site quality scores and/or mean exon coverage, particularly prior to the annotating.

3. The method of claim 1 or 2,
wherein selecting the subset of variant sites from the plurality of variant sites further comprises filtering the plurality of variant sites.

4. The method of claim 3, wherein the filtering is carried out prior to selecting the subset of variant sites in accordance with the predetermined gene set.

5. The method of any of the preceding claims, wherein predicting the presence and/or absence and/or an undetermined presence/absence of an alteration event at the respective variant site of the subset of variant sites is based on coverage information of the respective variant site.

6. The method of claim 5, wherein the predicting is further based on the annotations of the variant sites of the subset of variant sites and/or on data derived from the annotations of the variant sites of the subset of variant sites.

7. The method of any of the preceding claims, wherein the data derived from the annotations of the variant sites of the subset of variant sites are obtained by at least one of:
modifying the annotations by adding further annotation data retrieved from additional data sources, further filtering the subset of variant sites based on the annotations.

8. The method of any of the preceding claims, wherein predicting the presence and/or absence and/or undetermined presence/absence of an alteration event at the respective variant site of the subset of variant sites comprises determining a confidence score representative of a confidence for presence and/or absence and/or undetermined presence/absence of an alteration event at the respective variant site, in particular a false discovery rate, FDR, for an alteration event or an alteration non-event.

9. The method of claim 5 wherein the analysis data further comprises the determined confidence score.

10. The method of any of the preceding claims, wherein predicting presence and/or absence and/or undetermined presence/absence of an alteration event at the respective variant site is based on at least one of:
quality of RNA-sequencing data, particularly based on pre-determined sample inclusion and/or sample exclusion criteria,
quality of variant site, particularly determined using variant site quality scores,
mean exon coverage, particularly retrieved from a data source,
variant site depth and/or alternative allele coverage.

11. The method of any of the preceding claims, comprising providing (S170, S270, S370) the analysis data as input for further automated sample analysis, particularly a machine learning, ML-based analysis, and/or for use as machine learning training data for training an ML-based sample analysis tool and/or for reporting and/or visualization for supporting a user in a technical task.

12. The method of claim 11, wherein the providing of the analysis data comprises providing the annotations and/or the modified annotations and/or the final annotations and/or the predicted presence and/or absence and/or undetermined presence/absence of an alteration event at the respective variant site in the genome associated with the RNA sample.

13. The method of any of the preceding claims, wherein the gene set is application-specific, and optionally is selected taking into account information from the predetermined dataset.

14. The method of any of the preceding claims, wherein the predetermined dataset comprising the predetermined variant sites and associated annotation data is application-specific, in particular, comprises a set of predetermined variant sites classified as relevant for a predetermined tissue type and/or medical indication and/or detectable from RNA sequencing data.

15. The method of any of the preceding claims, comprising expanding (S180, S280) the predetermined dataset in response to encountering a variant site among the subset of variant sites that has no match in the predetermined data set, the modifying comprising annotating the variant site to obtain a new predetermined variant site and associated annotation data, and adding the new predetermined variant site and the associated annotation data to the predetermined data set.

16. Use of the annotation, particularly the final annotation, obtained by a method according to any of claims 1 to 15 for analyzing an RNA sample.

17. A system (100) configured to carry out the method of any of the preceding claims.

18. A computer program product comprising instructions which, when the program is executed by a computing system, cause the computing system to carry out the method of any of claims 1 to 16.

19. A computer-readable medium having stored thereon instructions which, when the program is executed by a computing system, cause the computing system to carry out the method of any of claims 1 to 16.
